# EUROPEAN PATENT SPECIFICATION

(11) **EP 2 051 687 B1**
(45) Date of publication and mention of the grant of the patent: **06.10.2010**
(21) Application number: 07808518.0
(22) Date of filing: 01.08.2007
(51) Int. Cl.: A61Q 19/00, A61K 8/06, A61K 8/41, A61K 8/81, A61K 8/365

(54) **EMULSION COMPOSITION FOR USE IN AN AFTER HAIR REMOVAL SKIN CARE PRODUCT**
EMULSIONSZUSAMMENSETZUNG ZUR VERWENDUNG IN EINEM PRODUKT ZUR PFLEGE DER HAUT NACH HAARENTFERNUNG
COMPOSITION D'ÉMULSION POUR UTILISATION DANS UN PRODUIT DE SOIN DE LA PEAU APRÈS ÉPILATION

(30) Priority: 01.08.2006 EP 06076514
(43) Date of publication of application: 29.04.2009
(73) Proprietor: Sara Lee/DE N.V., 3532 AA Utrecht (NL)
(72) Inventor: KLOMP, Andreas Joannes Anthonius, 2285 JE Rijswijk (NL); PRONK, Johannes, 2725 AM Zoetermeer (NL); PIESSENS, Josephus Petrus, 2275 BN Voorburg (NL); VOOIS, Karin, 2525 KG The Hague (NL); LAMBERS, Johannes Wilhelmus Jacobus, 2564 LB Den Haag (NL)
(74) Representative: Hatzmann, Martin
(86) International application number: PCT/NL2007/050385
(87) International publication number: WO 2008/016298

(56) References cited:
- EP-A2- 0 832 643
- WO-A1-00/27353
- WO-A2-01/85129
- US-A1- 2003 118 619
- DATABASE CAPLUS [Online] CHEMICAL ABSTRACTS SERVICE, COLUMBUS, OHIO, US; 18 May 2006 (2006-05-18), "Surfactant-free emulsions for skin containing glycyrrhizinic acid salts and moisturizers and their use as cosmetics" XP002415301 retrieved from STN Database accession no. 2006:466313 -& JP 2006 124319 A (NAPTEM SOKEN CO.) 18 May 2006 (2006-05-18)
- DATABASE WPI Week 197642 Derwent Publications Ltd., London, GB; AN 1976-78197X XP002415467 & JP 51 098341 A (KAWAKEN FINE CHEMI) 30 August 1976 (1976-08-30)

## Description

The present invention relates to skin care products for use after hair removal from the human face or body. The invention particularly relates to the use of an emulsion composition in an after hair-removal skin care product, such as an aftershave product, particularly in reducing and/or repairing skin damage resulting from mechanical and/or chemical processes such as shaving, peeling and depilation. The invention also relates to certain emulsion compositions as can be used in after hair-removal skin care products.

It is well-appreciated that skin shaving brings about the removal of several layers of skin, resulting in loss of skin barrier function which leads to drying out of the skin and to less resistance to colonization and easier penetration of potentially harmful micro-organisms. Hence, from medical, cosmetic as well as hygienic perspective, a rapid recovery of the barrier function of the skin is required. It is further observed that the skin needs to have a proper moisture content in order to have an adequate barrier function.

Object of the present invention is to provide an emulsion composition which allows for a rapid repair of the skin barrier function after the skin has been damaged by way of a mechanical and/or chemical process, whereas in addition it adequately moistures the skin.

Surprisingly, it has now been found that this can be realized when use is made of a particular combination of emulsion components.

Accordingly, the present invention relates to the use, for providing skin care after hair-removal, of an emulsion composition having a pH value in the range of from 4.0 to 7.0, which composition comprises a buffer, water, an oil in an amount in the range of from 3-20 wt%, a volatile nitrogen base or salt thereof, and a thickener in an amount in the range of from 0.5-3.0 wt%, whereby all weights are based on total emulsion composition.

The emulsion composition according to the present invention displays excellent skin barrier recovery and long-lasting skin moisturizing properties, whereas in addition it is highly stable. Further, the application of the emulsion composition onto the skin gives an enjoyable refreshing cool feeling.

Thus, the invention also pertains to the use of an emulsion composition as described above, for reducing and/or repairing skin damage that has been inflicted by means of a mechanical and/or a chemical process. Also, the invention pertains to the use of an emulsion composition as described in claim 1 for enhancing the skin barrier recovery, repair or restoration.

The emulsions provided serve skin care after hair removal (i.e. human face or body particularly after shaving, peeling-off hair, or depilation), and preferably are comprised in an aftershave product.

The thickener to be used in the emulsion composition according to the present invention is suitably chosen from the group consisting of mineral thickeners, natural and synthetic gums, synthetic polymers including copolymers and cross-polymers, and any chemical modifications thereof. Examples of mineral thickeners include bentonite and montmorillite. Examples of suitable natural or synthetic gums include gum arabic, gum tragacanth, cellulose gum, carraheenan, xanthan gum and their chemical modifications. Examples of suitable synthetic polymers include polyacrylate, dimethicon, vinyl, polyethylene, polypropylene glycol, and their chemical modifications.

The thickener to be used in accordance with the present invention preferably comprises an anionic polymer comprising counter-charges in the form of cationic groups that are derived from the volatile nitrogen base or salt thereof.

More preferably, the thickener is a polyacrylate or a copolymer or a cross-polymer of acrylate. Most preferably, the thickener is acryloyl dimethyltaurate/vinyl pyrrolidone copolymer.

The use of acryloyl dimethyltaurate/vinyl pyrrolidone copolymer as thickening agent and emulsion stabilizer is disclosed in US-A-2003/0118619 patent application.

The thickener to be used in accordance with the present invention is present in an amount in the range of from 0.5 to 3.0 wt%, based on total emulsion composition. Preferably, the thickener is present in an amount in the range of from 1.0 to 2.5 wt%, more preferably in an amount in the range of from 1.2 to 2.0 wt%, based on total emulsion composition.

The nitrogen base to be used is a volatile nitrogen base. In the context of the present invention this means that the nitrogen base has a boiling point below 200°C, preferably below 100°C, and more preferably below 65°C.

The volatile nitrogen base is preferably selected from the group consisting of ammonia, mono-ethanol amine, and alkyl amines of which the alkyl group comprises 1-4 carbon atoms. More preferably, the volatile nitrogen base is ammonia.

In accordance with the present invention the volatile nitrogen base can be present in the form of a salt. Suitable examples of such salts include, for instance, ammonium chloride, ammonium sulphate, ammonium lactate, and ammonium salts of the anionic polymer to be used as thickener as indicated hereinabove.

The volatile nitrogen base or its salt can suitably be present in an amount of from 0.02 to 0.50 mol per kg of the total emulsion composition.

Preferably, the volatile nitrogen base or its salt is present in an amount of from 0.05 to 0.25 mol per kg of the total emulsion composition.

The emulsion composition according to the present invention has a pH in the range of from 4.0 to 7.0. It should be understood that these pH values are the pH values of the emulsion composition as such, i.e. before it is being applied to skin.

In the present emulsion composition a buffer is present. Suitably, said buffer has in the pH range of from 5.0 to 7.0 a buffer capacity which is less than 0.5 times the molarity of the volatile nitrogen base or salt thereof used, whereas in the pH range of from 4.0 to 5.0 the buffer has a buffer capacity which is at least equal to and at most twice as high as the molarity of the volatile nitrogen base or the salt thereof used.

Preferably, said buffer has in the pH range of from 5.0 to 7.0 a buffer capacity which is less than 0.2 times the molarity of the volatile nitrogen base or the salt thereof used. More preferably, a buffer capacity which is less than 0.1 times the molarity of the nitrogen base or the salt thereof used.

Suitable examples of buffers to be used in accordance with the present invention include those selected from the group of consisting of mono- and di-carboxylic acids and their salts, and substituted mono- and di-carboxylic acids and their salts.

Preferably, the buffer is lactic acid/lactate or succinic acid/succinate.. Most preferably, the buffer is lactic acid/lactate.

In accordance with the present invention, the buffer is suitably present in an amount in the range of from 0.1 to 5.0 wt%, based on total emulsion composition. Preferably, the buffer is present in an amount in the range of from 0.5 to 2.5 wt%, based on total emulsion composition.

The amount of water in the emulsion composition according to the present invention can suitably be in the range of from 50-96 wt%, and is preferably in the range of from 65-85 wt%, based on total emulsion composition.

The particular combination of the components provides an emulsion composition that displays unique properties in terms of skin barrier recovery, skin moisturizing and a refreshing cool feel to the skin.

After application of the emulsion composition to skin, and due to the evaporation of the volatile nitrogen base and water, the pH of the skin will attractively have a value in the range of from 4.0 to 5.0. The latter pH range will facilitate an optimal recovery of the skin barrier function. In other words, the emulsion composition according to the present invention brings advantageously the pH of the skin to a desired value.

The emulsion composition according to the present invention has a surprisingly attractive and beneficial effect on the barrier function of the skin in one or more ways. In particular, the present emulsion composition has been found to have a strengthening effect on at least one of the following functions: (i) the physico-chemical barrier realised by the keratinocytes and the epidermal lipids forming bi-layered sheets embedding those keratinocytes typically found in the deeper layers of the Stratum Corneum, (ii) the microbiological barrier (at the surface of the Stratum Corneum), and (iii) proper homeostasis in order to maintain a strong, smooth, unbroken and flexible skin condition (in particular in the superficial layers of the Stratum Corneum).

In the skin, a pH gradient exists from the skin surface to the deepest layers. The pH at the surface is almost always lower than in the deeper parts (such as Stratum Compactum and Stratum Granulosum) where pH reaches that of the internal body fluids.

Without being bound to any theory, it is believed that the positive effect of the present emulsion on the barrier function of the skin, is partially due to the capacity to restore and/or maintain the pH of different layers of the skin at a normal (i.e. non-pathological) value.

The present emulsion composition according to the invention thus has been found to have a balancing effect on the skin, which helps to maintain the skin integrity.

The after hair-removal skin care products to which the invention pertains, preferably are distinguished from skin care products serving the reduction of fine lines, wrinkles, and other age-related effects. Such skin care products are generally based on retinol or retinoids, and are not after hair-removal skin care products. Preferably, the after hair-removal skin care products of the invention.are in essence free of retinol and retinoids. This avoids drawbacks may be associated with these substances, such as light instability and skin irritation which may occur to some extent (and which may be considered as a side-effect to be accepted in the treatment of fine lines, wrinkles or other age-related effects, but which is clearly undesirable in products serving the after hair removal skin care, such as aftershave products). In view of said possibility of skin irritation it is further preferred that the after hair removal skin care composition of the present invention in essence does not comprise alpha-hydoxy acid in a concentration higher than required for obtaining the above-mentioned buffer capacity.

Fragrances are also preferred rather than excluded. The invention also pertains to an advantageous embodiment in which no surfactant is required to provide a stable emulsion composition. Accordingly, the present emulsion composition is preferably in essence free of a surfactant.

Known emulsion compositions that are used after shaving to smooth the skin contain one or more emulsifiers. These emulsifiers are required to stabilize the oil droplets in the water-phase. Various emulsifiers and combinations of emulsifiers can be used including non-ionic surfactants / ethoxylates to prevent separation in multiple layers to occur. Draw back of such systems is its impact on the duration and the complexity of the production process and therefore increases costs. Moreover, surfactant free systems are less irritant to the skin.

In a preferred embodiment, the present invention provides an emulsion composition which is in essence free of emulsifiers including non-ionic surfactants and can be realized when use is made of a particular polymer which entraps the oil-droplets within its polymer matrix. I.e., the emulsion composition preferably contains less than 0.1 wt% emulsifier, based on total emulsion composition. Most preferably, the amount of surfactant present in the emulsion composition is less than 0.05 wt%, based on total emulsion composition.

Suitably, the present emulsion composition further comprises an astringent additive. Such an astringent additive tightens the skin. Suitably, the astringent additive is selected from the group consisting of metal salts, tannins and hazel extracts.

Preferably, the astringent additive is a soluble aluminium salt or a soluble zinc salt. More preferably, the astringent additive is alum or zinc sulphate.

The astringent additive is suitably present in an amount in the range of from 0.01 to 5.0 wt%, based on total emulsion composition. Preferably, the astringent additive is present in an amount in the range of from 0.1 to 1.0 wt%, based on total emulsion composition.

In addition, the emulsion composition can suitably comprise a fragrance. Any fragrance known to be used in body applications or aftershaves can be used for this purpose. The fragrance to be used is suitably present in an amount in the range of from 0.1-1.5 wt%, based on total emulsion composition. Preferably, the fragrance is present in an amount in the range of from 0.2 to 1.0 wt%, based on total emulsion composition.

Suitably, the emulsion composition in accordance with the present invention further comprises a humectant for promoting retention of moisture of the skin.

The humectant to be used in the present emulsion composition can suitably be selected from the group consisting of glycols, hydroxylated compounds, urea, pyrrolidine carboxylic acid, and any combinations thereof. Examples of suitable humectants include glycerol and sorbitol. Preferably, the humectant to be used in accordance with the present invention is glycerol.

Suitably, the humectant is present in an amount in the range of from 1.0-15.0 wt%, based on total emulsion composition. Preferably, the humectant is present in an amount in the range of from 2.0-6.0 wt%, based on total emulsion composition.

The oil to be used in the emulsion composition according to the present invention can suitably be selected from the group consisting of mineral and synthetic oils, vegetable oils and silicon-based oils.

The oil to be used is present in an amount in the range of from 3-20 wt%, based on total emulsion composition. Preferably, the oil is present in an amount in the range of from 5-20 wt%, and more preferably in an amount in the range of from 8-15 wt%, based on total emulsion composition.

In addition, the present emulsion composition may comprise any ingredients conventionally used in skin care compositions.

The emulsion composition according to the present invention can suitably be in the form of a cream, a lotion a paste or a gel. Preferably, the emulsion composition is present in the form of a lotion or cream, more preferably in the form of a lotion.

The emulsion composition can suitably have a viscosity in the range of from 5000-100000 cP (centipoises).

If applied in the form of a lotion, the emulsion composition will suitably have a viscosity in the range of from 5000-25000 cP, preferably in the range of 10000-20000 cP (as measured by Brookfield LVD, spindle 3, 6 rpm, 1 min., at 25°C).

If applied in the form of a cream, the emulsion composition will suitably have a viscosity in the range of from 25000-55000 cP, preferably in the range of 30000-45000 cP (as measured by Brookfield RVD, spindle 5, 5 rpm, 1 min., at 25°C).

The present invention also provides an after hair-removal skin care product comprising the emulsion composition according to claim 15.

In addition, the present invention provides an aftershave composition which comprises the emulsion composition according to the present invention.

Further, the present invention relates to the use of the present emulsion composition, the after hair-removal skin care composition, and particularly an aftershave composition according to the present invention for reducing and/or repairing skin damage that has been inflicted by means of a mechanical process and/or chemical process. Examples of mechanical and/or chemical processes include shaving, peeling and depilation. It will be understood shaving and depilation can apply to any part of the body, including face, head, armpits, legs and the like, whereas peeling or scrub will usually only be applied to the face.

In addition, the present invention relates to the use of the emulsion composition, the skin care composition or the aftershave composition according to the present invention for enhancing the skin barrier recovery, repair or restoration.

### Example

### PREPARATION OF AN AFTER HAIR-REMOVAL SKIN CARE COMPOSITION

A skin care composition in accordance with the present invention was prepared as follows. A first mixture was obtained by dissolving under stirring zinc sulphate (2.75 kg), glycerin (13.75 kg), caprylyl glycol (0.55 kg), lactic acid (0.55 kg), and sodium lactate (8.25 kg) in water (438.9 kg). A second mixture was obtained by mixing under stirring during 30 minutes ethylhexyl stearate (66.0 kg), a mixture of phenoxyethanol, ethyl paraben, methyl paraben and propyl paraben (5.5 kg in total), a perfume (2.75 kg) and ammonium acryloylmethyltaurate/vinyl pyrrolidone copolymer (11 kg). The second mixture was then added to the first mixture and the mixture so obtained was stirred for 20 minutes, whilst during 10 minutes a homogeniser was applied. In this way a cream was obtained.

### TESTING SKIN RECOVERY AND SKIN pH

The cream in accordance with the present invention was then used to help to repair the barrier of the tape-stripped skin of 10 subjects (individuals).

### TAPE-STRIP PROTOCOL

One spot (19x19 mm²) at the right forearm and one spot (19x19 mm²) at the left forearm were tape stripped with Pritt Sellotape Diamond ultra clear (Henkel) until Trans Epidermal Water Loss (TEWL) increase was in the range 15-25 g.m⁻².h⁻¹. The following procedure was used:
- A piece of tape was applied at skin;
- A preheated stainless steel weight (1000g, 64g/cm²; T=34°C) was applied for five seconds at the taped skin;
- The tape was removed by jerking parallel to the skin (as fast as possible);
- This procedure was repeated 15-30 times; and

### RECOVERY PERIOD

The barrier repair of the tape-stripped human skin was monitored during a period of nine days, both in respect of skin treated with the test product as well as untreated skin. During this period, the skin was soap-washed twice daily. This monitoring was done by measuring the TEWL values - values that are an excellent indicator for the quality of the skin's barrier.

### SOAP WASHING PROCEDURE

The inner sides of the forearms were washed after the tape stripping procedure on the evening of the first day, and subsequently on every morning and evening up to and including the morning of the ninth day. During the washing procedure the following protocol was used:
- The forearms were wetted with lukewarm tap water;
- During two minutes the forearms were washed by the subject with a 19% solution of natural soap bar (rubbing now and then);
- The forearms were then rinsed with tap water during about 15 seconds.
- The forearms were subsequently dried by means of dapping with a towel.

### PRODUCT APPLICATION PROTOCOL

The skin care composition in accordance with the present invention, and as described hereinbefore was applied at the tape-stripped skin by the subjects themselves. About 2 mg per cm² was applied every time. On the first day the composition was applied onto the skin after the last tape-stripping and after each soap washing, whereas on the second day up to and including the morning of the ninth day the composition was applied onto the skin after each soap washing.

### BIOPHYSICAL ASSESSMENTS

The TEWL values and skin pH values were then measured of the parts of skin treated as described hereinbefore. On the first day the values were measured before the treatment was started (baseline measurement), during the tape-stripping procedure (only TEWL values, data not shown), and 15 minutes after the soap washing treatment (start recovery measurement). Subsequently, the values were measured on the third, seventh and ninth day four hours after the skin care composition had been applied onto the skin. The TEWL values were measured by means of a Tewameter^{®} TM300 (Courage + Khazaka), whereas the pH value of the skin was measured by means of a Skin-pH-Meter^{®} PH 905 (Courage + Khazaka) 4 hours after application of the product. The subjects were 10 healthy volunteers of either sex who were aged between 18 and 65 years. The following conditions were applied during the measurements: a temperature of 22° C (standard deviation of 1°C); a relative humidity of 45% (standard deviation of 5%); an equilibration period of 30 minutes. In the statistical analysis a two-tailed Paired Student's t-test was applied and a critical p-value (probability-value) for significance of 0.05 was used. In the data obtained the recovery is expressed as (1 - "TEWL increase" / "initial TEWL increase")*100%, whereas the TEWL increase is defined as the TEWL value of tape stripped skin minus the TEWL value of the intact skin. In other words, both the two tape-stripped skin fields as well as the intact (soap-washed) skin field were measured.

### RESULTS

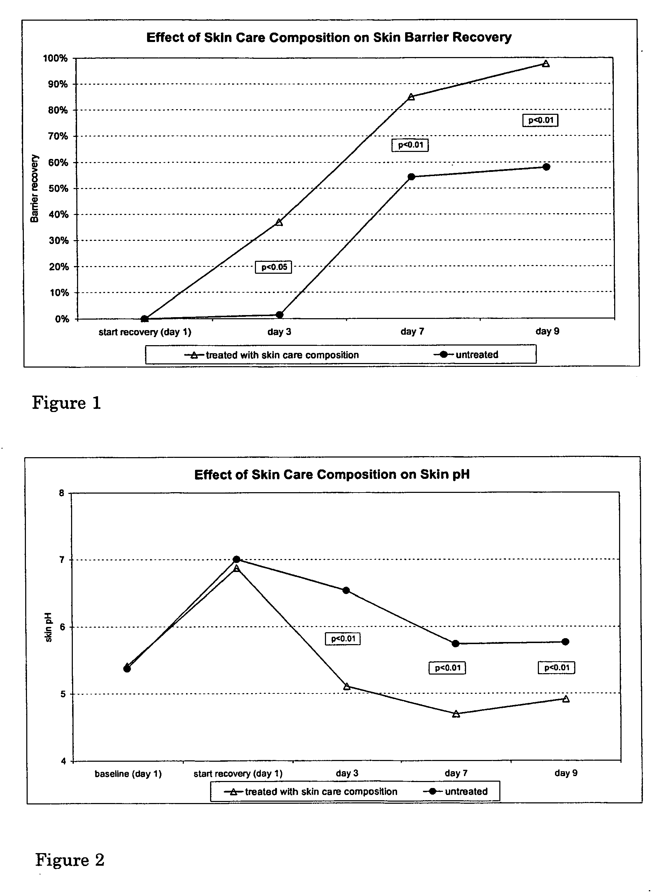

From the results shown in Figure 1, it will be clear that the application of the present skin care composition onto tape-stripped human skin resulted in a significantly quicker recovery of the barrier of the skin (see figure 1), when compared with untreated skin. In addition, the use of the composition in accordance with the present invention also resulted in a significant lower skin pH (see Figure 2).

## Claims

1. The use for providing skin care after hair-removal, of an emulsion composition, preferably in an aftershave product, having a pH value in the range of from 4.0 to 7.0, which composition comprises a buffer, water, an oil in an amount in the range of from 3 to 20 wt%, a volatile nitrogen compound or salt thereof, and a thickener in an amount in the range of from 0.5 to 3 wt%, whereby all weights are based on total emulsion composition.

2. The use of an emulsion composition as described in claim 1 for reducing and/or repairing skin damage that has been inflicted by means of a mechanical and/or a chemical process.

3. The use of an emulsion composition as described in claim 1 or 2 for enhancing the skin barrier recovery, repair or restoration.

4. A use according to any one of the preceding claims, wherein the thickener comprises an anionic polymer comprising counter charges in the form of cationic groups that are derived from the volatile nitrogen base or the salt thereof, preferably a polyacrylate, or a co-polymer or cross-polymer of an acryl ate.

5. A use according to any one of the preceding claims, wherein the thickener is present in an amount in the range of from 1.0 to 2.5 wt%, based on total emulsion composition.

6. A use according to any one of the preceding claims, wherein the volatile nitrogen base or salt thereof is present in an amount in the range of from 0.02 to 0.5 mol per kg of the total emulsion composition.

7. A use according to any one of the preceding claims, wherein the volatile nitrogen base is selected from the group consisting of ammonia, mono-ethanol amine, and alkyl amines of which the alkyl group comprises 1-4 carbon atoms.

8. A use according to any one of the preceding claims, wherein the composition has a pH in the range of from 5.0 to 6.5.

9. A use according to any one of the preceding claims, wherein the buffer has in the pH range of from 5.0 to 7.0 a buffer capacity which is less than 0.5 times, and preferably less than 0.2 times, the molarity of the volatile nitrogen base or salt thereof, whereas in the pH range of from 4.0 to 5.0 the buffer has a buffer capacity which is at least equal to and at most twice as high as the molarity of the volatile nitrogen base or the salt thereof.

10. A use according to any one of the preceding claims, wherein the buffer is selected from the group of consisting of mono- and di-carhoxylic acids and their salts, and substituted mono- and di-carboxylic acids and their salts, preferably lactic acid/lactate or succinic acid/succinate.

11. A use according to any one of the preceding claims, wherein water is present in the emulsion in an amount in the range of from 50-96 wt%, based on total emulsion composition.

12. A use according to any one of the preceding claims, wherein the composition further comprises an astringent additive, preferably selected from the group consisting of metal salts, preferably a soluble aluminium salt or a soluble zinc salt, tannins and hazel extracts, in an amount in the range of from 0.01-5.0 wt%, based on total emulsion composition.

13. A use according to any one of the preceding claims, wherein the composition further comprises a fragrance in an amount in the range of from 0.1-1.5 wt%, based on total emulsion composition.

14. A use according to any one of the preceding claims, wherein the composition further comprises a humectant for promoting retention of moisture of the skin, preferably selected from the group consisting of glycols, preferably glycerol, hydroxylated compounds, urea, pyrrolidine carboxylic acid, and any combinations thereof, in an amount in the range of from 1.0-15.0 wt%, based on total emulsion composition.

15. An after hair-removal skin care composition comprising an emulsion composition as used in any one of the preceding claims, the emulsion containing less than 0.1 wt.%, preferably less than 0.05 wt.%, emulsifier, wherein the emulsion contains a polymer matrix in which the oil has been entrapped, the polymer being acryloyl dimethyltaurate/vinyl pyrrolidone copolymer.

## Patentansprüche

1. Die Verwendung einer Emulsionszusammensetzung zum Pflegen der Haut nach der Haarentfernung, bevorzugt in einem AfterShave-Produkt, mit einem pH-Wert im Bereich von 4,0 bis 7,0, wobei die Zusammensetzung einen Puffer, Wasser, ein Öl in einer Menge im Bereich von 3 bis 20 Gew.-%, eine flüchtige Stickstoffverbindung oder ein Salz davon und ein Verdickungsmittel in einer Menge im Bereich von 0,5 bis 3 Gew.-% umfasst, wobei alle Gewichte sich auf die Gesamtemulsionszusammensetzung beziehen.

2. Die Verwendung einer Emulsionszusammensetzung, wie sie in Anspruch 1 beschrieben ist, zur Reduzierung und/oder Reparatur von Hautschäden, die durch einen mechanischen und/oder einen chemischen Prozess verursacht wurde.

3. Die Verwendung einer Emulsionszusammensetzung, wie sie in Anspruch 1 oder 2 beschrieben ist, zur Verbesserung der Erholung, Reparatur oder Wiederherstellung der Hautbarriere.

4. Eine Verwendung nach irgendeinem der vorhergehenden Ansprüche, wobei das Verdickungsmittel ein anionisches Polymer umfasst, das Gegenladungen in der Form kationischer Gruppen umfasst, die von der flüchtigen Stickstoffbase oder deren Salz abgeleitet sind, bevorzugt ein Polyacrylat oder ein Copolymer oder Kreuzpolymer eines Acrylats.

5. Eine Verwendung nach irgendeinem der vorhergehenden Ansprüche, wobei das Verdickungsmittel in einer Menge im Bereich von 1,0 bis 2,5 Gew.-% bezogen auf die Gesamtemulsionszusammensetzung vorhanden ist.

6. Eine Verwendung nach irgendeinem der vorhergehenden Ansprüche, wobei die flüchtige Stickstoffbase oder deren Salz in einer Menge im Bereich von 0,02 bis 0,5 Mol pro kg der Gesamtemulsionszusammensetzung vorhanden ist.

7. Eine Verwendung nach irgendeinem der vorhergehenden Ansprüche, wobei die flüchtige Stickstoffbase aus der Gruppe ausgewählt wird, die aus Ammoniak, Monoethanolamin und Alkylaminen besteht, deren Alkylgruppe 1-4 Kohlenstoffatome umfasst.

8. Eine Verwendung nach einem der vorhergehenden Ansprüche, wobei die Zusammensetzung einen pH im Bereich von 5,0 bis 6,5 aufweist.

9. Eine Verwendung nach einem der vorhergehenden Ansprüche, wobei der Puffer im pH-Bereich von 5,0 bis 7,0 eine Pufferkapazität aufweist, die weniger als das 0,5-fache, und bevorzugt weniger als das 0,2-fache der Molarität der flüchtigen Stickstoffbase oder deren Salz beträgt, während der Puffer im pH-Bereich von 4,0 bis 5,0 eine Pufferkapazität aufweist, die der Molarität der flüchtigen Stickstoffbase oder deren Salz wenigstens gleich ist und höchstens das Doppelte davon beträgt.

10. Eine Verwendung nach einem der vorhergehenden Ansprüche, wobei der Puffer aus der Gruppe ausgewählt ist, die aus Mono- und Dicarbonsäuren und deren Salzen und substituierten Mono- und Dicarbonsäuren und deren Salzen, bevorzugt Milchsäure/Lactat oder Bernsteinsäure/Succinat besteht.

11. Eine Verwendung nach irgendeinem der vorhergehenden Ansprüche, wobei Wasser in einer Menge im Bereich von 50 bis 96 Gew.-% bezogen auf die Gesamtemulsionszusammensetzung vorhanden ist.

12. Eine Verwendung nach irgendeinem der vorhergehenden Ansprüche, wobei die Zusammensetzung ferner ein adstringierendes Additiv, das bevorzugt aus der Gruppe ausgewählt ist, die aus Metallsalzen, bevorzugt einem löslichen Aluminiumsalz oder einem löslichen Zinksalz, Tanninen und Haselnussextrakten besteht, in einer Menge im Bereich von 0,01-5,0 Gew.-% bezogen auf die Gesamtemulsionszusammensetzung umfasst.

13. Eine Verwendung nach irgendeinem der vorhergehenden Ansprüche, wobei die Zusammensetzung ferner einen Duftstoff in einer Menge im Bereich von 0,1-1,5 Gew.-% bezogen auf die Gesamtemulsionszusammensetzung umfasst.

14. Eine Verwendung nach irgendeinem der vorhergehenden Ansprüche, wobei die Zusammensetzung ferner ein Feuchthaltemittel zur Förderung der Feuchtigkeitsspeicherung der Haut, das bevorzugt aus der Gruppe ausgewählt ist, die aus Glykolen, bevorzugt Glycerol, hydroxylierten Verbindungen, Harnstoff, Pyrrolidincarbonsäure und beliebigen Kombinationen daraus besteht, in einer Menge von 1,0-15,0 Gew.-% bezogen auf die Gesamtemulsionszusammensetzung umfasst.

15. Eine nach der Haarentfernung eingesetzte Hautpflegezusammensetzung, die eine Emulsionszusammensetzung umfasst, wie sie in irgendeinem der vorhergehenden Ansprüche verwendet wird, wobei die Emulsion weniger als 0,1 Gew.-%, bevorzugt weniger als 0,05 Gew.-% eines Emulgators enthält, wobei die Emulsion eine Polymermatrix enthält, in die das Öl eingeschlossen wurde, wobei das Polymer ein Acryloyldimethyltaurat/Vinylpyrrolidon Copolymer ist.

## Revendications

1. Utilisation pour fournir un soin de la peau après épilation, d'une composition d'émulsion, de préférence dans un produit après-rasage, ayant une valeur de pH dans la fourchette comprise entre 4,0 et 7,0, ladite composition comprenant un tampon, de l'eau, une huile en une quantité dans la fourchette comprise entre 3 et 20 %, un composé azoté volatil ou un sel de celui-ci, et un épaississant, en une quantité dans la fourchette comprise entre 0,5 et 3 % en poids, dans laquelle tous les poids sont basés sur la composition totale de l'émulsion.

2. Utilisation d'une composition d'émulsion telle que décrite selon la revendication 1, pour réduire et/ou réparer l'endommagement de la peau, ayant été infligé au moyen d'un processus mécanique et/ou d'un processus chimique.

3. Utilisation d'une composition d'émulsion telle que décrite selon la revendication 1 ou 2, pour améliorer la récupération, la réparation ou la restauration de la fonction de barrière assurée par la peau.

4. Utilisation selon l'une quelconque des revendications précédentes, dans laquelle l'épaississant comprend un polymère anionique, comprenant des contre-charges se présentant sous la forme de groupes cationiques dérivés de la base azotée volatile ou du sel de celle-ci, de préférence un polyacrylate, ou un copolymère ou un polymère croisé d'un acrylate.

5. Utilisation selon l'une quelconque des revendications précédentes, dans laquelle l'épaississant est présent en une quantité dans la fourchette comprise entre 1,0 et 2,5 % en poids, basés sur la composition totale de l'émulsion.

6. Utilisation selon l'une quelconque des revendications précédentes, dans laquelle la base azotée volatile ou le sel de celle-ci est présent(e) en une quantité dans la fourchette comprise entre 0,02 et 0,5 mole par Kg de la composition totale de l'émulsion.

7. Utilisation selon l'une quelconque des revendications précédentes, dans laquelle la base azotée volatile est sélectionnée dans le groupe composé du gaz ammoniac, de la mono-éthanol amine, et des alkyl amines, dont le groupe alkyle comprend de 1 à 4 atomes de carbone.

8. Utilisation selon l'une quelconque des revendications précédentes, dans laquelle la composition présente un pH dans la fourchette comprise entre 5,0 et 6,5.

9. Utilisation selon l'une quelconque des revendications précédentes, dans laquelle le tampon présente un pH dans la fourchette comprise entre 5,0 et 7,0, une capacité tampon inférieure à 0,5 fois et, de préférence, inférieure à 0,2 fois, la molarité de la base azotée volatile ou de son sel, tandis que, dans la fourchette de pH allant de 4,0 à 5,0, le tampon présente une capacité tampon au moins égale à, et au plus du double de, la molarité de la base azotée volatile ou de son sel.

10. Utilisation selon l'une quelconque des revendications précédentes, dans laquelle le tampon est sélectionné dans le groupe composé des acides mono- et dicarboxyliques et leurs sels, et d'acides mono- et dicarboxyliques substitués et leurs sels, de préférence acide lactique/lactate ou acide succinique/succinate.

11. Utilisation selon l'une quelconque des revendications précédentes, dans laquelle de l'eau est présente dans l'émulsion, en une quantité dans la fourchette comprise entre 50 et 96 % en poids, basés sur la composition totale de l'émulsion.

12. Utilisation selon l'une quelconque des revendications précédentes, dans laquelle la composition comprend en outre un additif astringent, de préférence sélectionné dans le groupe composé de sels métalliques, de préférence un sel d'aluminium soluble ou un sel de zinc soluble, des extraits de tanins et de noisetier, en une quantité dans la fourchette comprise entre 0,01 et 5,0 % en poids, basés sur la composition totale de l'émulsion.

13. Utilisation selon l'une quelconque des revendications précédentes, dans laquelle la composition comprend en outre un parfum, en une quantité dans la fourchette comprise entre 0,1 et 1,5 % en poids, basés sur la composition totale de l'émulsion.

14. Utilisation selon l'une quelconque des revendications précédentes, dans laquelle la composition comprend en outre un agent de mouillage, pour favoriser la rétention de l'humidité de la peau, de préférence sélectionné dans le groupe composé des glycols, de préférence glycérine, composés hydroxylatés, urée, acide pyrrolidine-carboxylique, et toutes combinaisons de deux-ci, en une quantité dans la fourchette comprise entre 1,0 et 15,0 % en poids, basés sur la composition totale de l'émulsion.

15. Composition de soin de la peau après épilation, comprenant une composition d'émulsion telle qu'utilisée selon l'une quelconque des revendications précédentes, l'émulsion contenant moins de 0,1 % en poids, de préférence moins de 0,05 % en poids d'émulsifiant, dans laquelle l'émulsion contient une matrice de polymère dans laquelle l'huile a été emprisonnée, le polymère étant un copolymère acryloyl diméthyltaurate/vinylpyrrolidone.
